# EUROPEAN PATENT SPECIFICATION

(11) **EP 4 486 290 B1**
(45) Date of publication and mention of the grant of the patent: **03.12.2025**
(21) Application number: 23706795.4
(22) Date of filing: 27.02.2023
(51) Int. Cl.: A61K 8/37, A61Q 5/00, A61Q 19/00, C11D 3/20, C11D 3/50, C11D 7/26, C11D 11/00

(54) **FIXATIVE MOLECULES**
FIXIERUNGSMOLEKÜLE
MOLÉCULES FIXATIVES

(30) Priority: 01.03.2022 WO PCT/EP2022/055126
(43) Date of publication of application: 08.01.2025
(73) Proprietor: Symrise AG, 37603 Holzminden Niedersachsen (DE)
(72) Inventor: IMPERIALE, Michael V., KEARNY, New Jersey 07032 (US); MCDERMOTT, Keith, BOUND BROOK, New Jersey 08805 (US); PONZONI, Joseph, WASHINGTONVILLE, New York 10992 (US)
(74) Representative: Eisenführ Speiser
(86) International application number: PCT/EP2023/054783
(87) International publication number: WO 2023/165915

(56) References cited:
- CN-B- 103 750 264
- JP-A- 2009 150 036
- US-A1- 2012 142 578

## Description

The present invention relates to a method for imparting a fragrance to a fabric comprising the steps: (i) providing a composition comprising or consisting of one or more fragrance compound(s) and at least one polyol ester of the formula (I) as defined below; (ii) contacting the composition from step (i) with the fabric; and (iii) optionally, drying the fabric. Furthermore, the present invention provides a fragrant object obtained or obtainable by the method according to the invention, a composition comprising or consisting of one or more fragrance compound(s) and at least one polyol ester of the formula (I) as defined below, a product comprising a composition according to the invention as well as the use of a polyol ester of the formula (I) as defined below for enhancing deposition of a fragrance on a fabric.

By definition and function, rinse-off products for laundry or personal care use do not effectively deposit fragrance onto substrates such as clothing (synthetic or natural) and human skin. Classically, some of the fragrance is deposited, while the bulk is rinsed away with the product during use. A person skilled in the art of perfumery can design a fragrance to be inherently substantive, but is limited in a few olfactive spaces. By depositing more fragrance on the substrate, there is an increase in: fragrance strength, fragrance quality and any and all ancillary benefits a fragrance imparts, including but not limited to malodor counteraction activity, cosmetic benefit and/or antimicrobial properties.

While microencapsulation technology provides improved deposition of fragrance onto textiles, fragrance microcapsules can be cost prohibitive, limit hedonic directions due to interactions with capsule shell wall material, and pose a micro-plastics concern.

The advantages of depositing more fragrance are clearly beneficial to the consumer in that increased fragrance longevity, intensity and hedonic profile are key drivers of purchase and re-purchase intent and provide superior in-use experiences.

US9701929B2 discloses organopolysiloxane emulsions containing a benefit agent, e.g. a fragrance. The emulsion facilitates increased deposition and retention of the benefit agent onto a target surface such as fabric, skin or hair.

US patent US7776347B2 relates to a structured premix or "delivery vehicle" composition for personal care products, which is designed as a carrier to enhance - among others - the benefit of a hydrophobic benefit agent, such as perfume, via enhanced deposition. The hydrophobic benefit agent may be entrapped within a network structure formed by the premix composition. The structuring material may be e.g. a crystalline wax, hydrogenated oil or fat.

US7524807B2 describes a rinse-off personal care composition comprising a perfume polymeric particle, which is useful as a delivery system for a perfume raw material to skin or hair. The perfume polymeric particle comprises an anionic polymer and a perfume. A polymeric encapsulated fragrance for personal care and cleaning products is disclosed in US7491695B2. Polyacrylate microcapsules are used according to US9561169B2 in a conditioner to increase deposition of a benefit agent such as a perfume on hair.

US6852681B1 relates to a perfume-containing bar composition with enhanced longevity of the perfume and a process to for enhancing deposition/longevity of a perfume. The deposition/longevity is enhanced according to US6852681B1 by keeping the amount of soluble surfactants below a maximum 35% by wt.

US7749952B2 discloses a fabric care composition which contains glyceryl triesters as fabric care softening agents. Additionally, this patent discloses a composition that contains sucrose esters and polymeric primary and secondary amines as perfume deposition aids for enhanced perfume longevity.

JP2009150036 describes glycerine triesters as having improved fragrance rate of absorption onto clothing.

CN1 03750264B discloses a preparation and use of glycerine monoester of C8 chain length within a microcapsule application to modulate vapor pressure of aroma molecules as it relates to aroma molecule evaporation from a substrate such as meat.

US 2012/0142578 relates to a fabric care composition comprising a mixture of glycerides and a delivery enhancing agent, for providing a benefit to a fabric by contacting the fabric with said fabric care composition.

Despite previous efforts to increase deposition of fragrances on substrates, there is still a need for novel methods and compositions, which are capable, in particular, to enhance the deposition of fragrances, which are notoriously difficult to deposit.

It was therefore an objective of the present invention to provide compositions, in particular in the form of rinse-off products, which allow to deposit significant amounts of fragrance(s) on fabric and thus allow to take better advantage of the beneficial properties of the fragrance(s) including - besides the fragrance - also e.g. antimicrobial properties.

In particular, the compositions should be able to impart long lasting fragrance to fabric during operations such as washing, wherein the fragrance is not significantly lost during rinsing or drying.

The compositions should also be able to deposit significant amounts of difficult to deposit fragrances.

The above objectives are met by a method for imparting a fragrance to a fabric comprising or consisting of the following steps:
(i) providing a composition comprising or consisting of one or more fragrance compound(s) and at least one polyol ester of the formula (I) 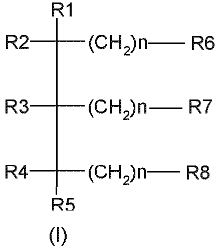
   wherein R1, R2, R4 and R5 are independently selected from H, methyl and ethyl, and R3 is methyl or ethyl, preferably ethyl,
   each n is independently 0, 1 or 2,
   R6 to R8 are independently selected from H, OH, R9COO- and R10COO-,
   wherein R9 is a branched or linear, saturated or unsaturated alkyl group with 8 to 10 carbon atoms, and
   at least two of R6 to R8 are R10COO-,
   wherein R10 is a linear, saturated alkyl with 8 to 10 carbon atoms,
   preferably, wherein R9 is a linear, saturated or unsaturated alkyl group with 8 to 10 carbon atoms,
   preferably, wherein R6 and R7 are R10COO- and R8 is R9COO-,
(ii) contacting the composition from step (i) with the fabric, and
(iii) optionally, drying the fabric.

Polyol esters are hydrophobic, oil soluble compounds. It was found out that, when dosed into a fragrance, which is subsequently dosed into a consumer rinse off-product, certain polyol esters deposit more fragrance onto fabric than a control sample without an added polyol ester. This novel and unexpected discovery provides a new mechanism of fragrance fixation beyond state-of-the art methods, known by those skilled in the art and science of consumer fragrance design. Thus far, the polyol ester as defined herein has not been brought in conjunction with enhanced deposition of a fragrance on a fabric, let alone with its degree of its enhancement of depositing a fragrance.

Polyol esters provide advantages over state of the art deposition aids/fixatives since they are: miscible with hydrophobic phases and minimally partition into the aqueous phase or require an aqueous medium, like most polyquats. Additionally, this class of molecules does not impart harsh pH environments which can cause, for example, hydrolysis of fragrant esters. Fragrance deposition is not selective in that only certain types of fragrance ingredients deposit, but rather, the increase in deposition is representative of notes that are not expected to present on dry fabric; this opens new creative opportunities for fragrance construction and enables new creative possibilities for dry fabric performance. Dry fabric performance, using current state of the art deposition technologies, is limited in that, generally speaking, mostly base notes and some mid notes deposit, limiting hedonic profiles in this stage. However, with the inclusion of polyol esters as defined herein to a fragrance, it has been observed sensorially and measured analytically that top, mid and base notes have greater deposition on dry fabric.

Structurally, polyol esters are formed by reacting fatty acids with a polyol such as glycol. Corresponding methods are known to the skilled person. In addition, polyol esters of formula (I) and (II) are available commercially.

In one embodiment of the method described above, in the polyol ester of formula (I) R1, R2, R4 and R5 are H.

In another embodiment of the method according to any of the embodiments described above, two n are 0 and one n is 1. Preferably, each n is 1.

In the most preferred embodiment of the method according to any of the embodiments described above, R3 is CH₂CH₃.

In one embodiment of the method described above, the polyol ester has the formula (II) 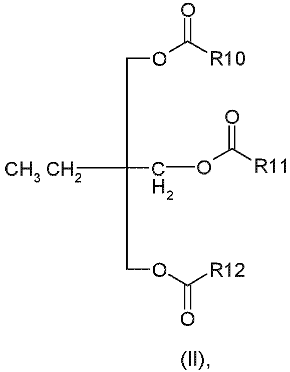 wherein R10 to R12 are independently selected from linear, saturated alkyl with 8 to 10 carbon atoms.

Preferably, the fabric is selected from the group consisting of cotton and synthetic fabrics, in particular polyester, spandex, nylon and lycra and mixtures thereof.

As can be inferred from the examples below, the method of the present invention can impart a long lasting fragrance on different fabrics when added to the laundry, which also remains during air drying or machine drying in a tumble dryer. Thus, fragrance can be imparted e.g. on clothes, towels and bed sheets and other materials and/or textiles made of cotton, synthetic or blends thereof. In some embodiments, the method does not involve the use of, and/or the fabric is free of, starch adjuvants.

"Contacting" in the context of the present invention therefore refers in particular to a washing process of fabric, either washing by hand or in a washing machine, in which the composition of step (i) is added to the washing liquid and present throughout at least part of the washing process. After rinsing to remove the soap, the fragrance remains deposited to a large extent even when using a dryer at higher temperatures. Preferably, the composition of step (i) is contacted with fabric by incorporating it into a liquid laundry-care application selected from the group consisting of laundry detergent, liquid laundry detergent, fabric softener, liquid fabric softener and combination thereof.

In one embodiment of the method described above, in step (ii) the composition from step (i) is added to water, optionally including soap and/or fabric softener, in which the fabric is soaked.

In one embodiment of the method according to any of the embodiments described above, the composition additionally imparts conditioning and/or softening effects on the fabric.

Besides imparting a fragrance, the composition provided in step (i) of the method according to any of the embodiments described above, can also provide conditioning and/or softening effects on fabric. Thus, further addition of conditioner or fabric softener can be avoided.

In one embodiment of the method described above, the composition provided in step (i) has a weight ratio of 50:50 or less polyol ester(s) to fragrance compound(s), preferably 25:75 or less, particularly preferably 10:90 or less. In a preferred embodiment of the method described in any of the embodiments above, the composition provided in step (i) has a weight ratio in a range of 9:91 to 1:99 polyol ester(s) to fragrance compound(s), preferably in a range of 6:94 to 1.5:98.5.

The present invention allows to efficiently deposit a wide range of fragrances. In particular, the fragrance compounds are such compounds, which are commonly used or designed for liquid laundry detergent or fabric softener applications.

In one embodiment of the method described above, the fragrance compound(s) is/are selected from aldehydes, ketones, esters, nitriles, ethers, lactones, primary alcohols, secondary alcohols, tertiary alcohols, phenolics, hydrocarbons, acetals and essential oils. Some very desirable fragrance compounds or mixtures are particularly difficult to deposit on a fabric in an amount that imparts advantageous effects such as fragrance and antimicrobial properties. These compounds or mixtures of compounds are often quickly lost or only base and mid notes remain on the fabric, while top notes are lost changing the fragrance impression to a less complex and often less desirable one. Thus, the quality of the fragrance is low. Advantageously, using the method of the present invention, it is also possible to deposit such fragrance compounds or mixtures thereof efficiently and to impart a long lasting fragrance, which does not change significantly over time.

Therefore, in one embodiment of the method according to any embodiments described above, the fragrance compound(s) is/are selected from the group consisting of compounds listed in the following Table 1.

**Table 1:**

| **Perfume Raw Material** | **CAS** | **IUPAC Name** |
|---|---|---|
| AGRUNITRIL | 51566-62-2 | 3,7-dimethyl-6-octenenitrile |
| ALDEHYDE C11 MOA PURE | 19009-56-4 | 2-Methyl Decanal |
| ALDEHYDE C11 UNDECYLENIC | 112-45-8 | 10-Undecanal |
| ALDEHYDE C11 UNDECYLIC | 112-44-7 | Undecanal |
| ALDEHYDE C12 LAURIC | 112-54-9 | Dodecanal |
| ALDEHYDE C12 MNA | 110-41-8 | 2-Methyl undecanal |
| ALDEHYDE C14 SO-CALLED | 104-67-6 | 5-heptyloxolan-2-one |
| AMBER CORE | 139504-68-0 | 1-(2-tert-butylcyclohexyl)oxybutan-2-ol |
| AMBERWOOD^{®} F | 58567-11-6 | ethoxymethoxycyclododecane |
| AMBROCENIDE^{®} 10 DPG | 211299-54-6 | (1R,8R,10S,13R)-5,5,7,9,9,13-hexamethyl-4,6-dioxatetracyclo[6.5.1.01,10.03,7]tetradecane |
| AMBROXIDE | 6790-58-5 | (3aR,5aS,9aS,9bR)-3a,6,6,9a-tetramethyl-2,4,5,5a,7,8,9,9b-octahydro-1H-benzo[e][1]benzofuran |
| AMYL CINNAMIC ALDEHYDE ALPHA | 122-40-7 | (2Z)-2-benzylideneheptanal |
| AMYL SALICYLATE | 2050-08-0 | pentyl 2-hydroxybenzoate |
| ANETHOL SUPRA 21,5 CELSIUS | 4180-23-8 | 1-methoxy-4-[(E)-prop-1-enyl]benzene |
| BELANIS^{®} | 1447712-18-6 | 2-methyl-5-propylcyclohex-2-en-1-one |
| BENZYL SALICYLATE | 118-58-1 | benzyl 2-hydroxybenzoate |
| BETA NAPTHYL ISO BUTYL ETHER | 2173-57-1 | 2-isobutoxynaphthalene |
| CEDARWOOD OIL VIRGINIA | 8000-27-9 | ESSENTIAL OIL - CEDROL |
| CEDRAMBER | 67874-81-1 | (1S,2R,5S,7R,8R)-8-methoxy-2,6,6,8-tetramethyltricyclo[5.3.1.01,5]undecane |
| CITRONELLOL 950 | 106-22-9 | 3,7-dimethyloct-6-en-1-ol |
| CITRONELLYL ACETATE EXTRA | 150-84-5 | 3,7-dimethyloct-6-enyl acetate |
| CITRONELLYL BUTYRATE | 141-16-2 | 3,7-dimethyloct-6-enyl butanoate |
| CITRONELLYL ISOBUTYRATE | 97-89-2 | 3,7-dimethyloct-6-enyl 2-methylpropanoate |
| CITRONELLYL PROPIONATE | 141-14-0 | 3,7-dimethyloct-6-enyl propanoate |
| CITRONITRILE | 93893-89-1 | 3-Methyl-5-phenylpent-2-enenitrile |
| CLONAL | 2437-25-4 | Dodecanenitrile |
| CYCLABUTE | 68039-39-4 | 8-tricyclo[5.2.1.02,6]dec-3-enyl 2-methylpropanoate |
| CYCLAMEN ALDEHYDE | 103-95-7 | 2-methyl-3-(4-propan-2-ylphenyl)propanal |
| CYCLOHEXYL SALICYLATE | 25485-88-5 | cyclohexyl 2-hydroxybenzoate |
| DAMASCENONE | 23726-93-4 | (E)-1-(2,6,6-trimethylcyclohexa-1,3-dien-1-yl)but-2-en-1-one |
| DAMASCONE ALPHA | 24720-09-0 | (E)-1-(2,6,6-trimethylcyclohex-2-en-1-yl)but-2-en-1-one |
| DAMASCONE BETA | 35044-68-9 | 1-(2,6,6-trimethylcyclohexen-1-yl)but-2-en-1-one |
| DAMASCONE DELTA | 71048-82-3 | 1-(2,6,6-trimethylcyclohex-3-en-1-yl)but-2-en-1-one |
| DIMETHYL ANTHRANILATE EXTRA | 85-91-6 | Methyl-2-(methylamino)benzoate |
| EBANOL | 67801-20-1 | 3-Methyl-5-(2,2,3-trimethyl-3-cyclopenten-1-yl)-4-penten-2-ol |
| ETHYL LINALOOL | 10339-55-6 | (6E)-3,7-dimethylnona-1,6-dien-3-ol |
| ETHYL SALICYLATE | 118-61-6 | ethyl 2-hydroxybenzoate |
| FLORALOZONE | 67634-15-5 | 3-(4-ethylphenyl)-2,2-dimethylpropanal |
| GERANIOL SUPER | 106-24-1 | (2E)-3,7-dimethylocta-2,6-dien-1-ol |
| GERANYL ACETATE 60 | 105-87-3 | [(2E)-3,7-dimethylocta-2,6-dienyl] acetate |
| GERANYL ISOBUTYRATE | 2345-26-8 | [(2E)-3,7-dimethylocta-2,6-dienyl] 2-methylpropanoate |
| GERANYL PROPIONATE | 105-90-8 | [(2E)-3,7-dimethylocta-2,6-dienyl] propanoate |
| GLOBALIDE^{®} | 34902-57-3 | (3E)-1-oxacyclohexadec-3-en-2-one |
| HEDIONE | 24851-98-7 | methyl 2-(3-oxo-2-pentylcyclopentyl)acetate |
| HERBAFLORAT | 5413-60-5 | 8-tricyclo[5.2.1.02,6]dec-3-enyl acetate |
| HERBYL PROPIONATE | 68912-13-0 | 3-tricyclo[5.2.1.02,6]dec-4-enyl propanoate |
| HEXYL BENZOATE | 6789-88-4 | hexyl benzoate |
| HEXYL CINNAMIC ALDEHYDE ALPHA | 165184-98-5 | (2E)-2-benzylideneoctanal |
| HEXYL SALICYLATE | 6259-76-3 | hexyl 2-hydroxybenzoate |
| ISO E SUPER NON DISCOLORING | 54464-57-2 | 1-(2,3,8,8-tetramethyl-1,3,4,5,6,7-hexahydronaphthalen-2-yl)ethanone |
| ISOAMYL SALICYLATE | 87-20-7 | 3-methylbutyl 2-hydroxybenzoate |
| ISOBUTYL SALICYLATE | 87-19-4 | 2-methylpropyl 2-hydroxybenzoate |
| ISOLONGIFOLANON COEUR | 29461-14-1 | (3S,6S)-2,2,8,8-tetramethyl-octahydro-1H-2,4a-methanonapthalen-10-one |
| KEPHALIS | 36306-87-3 | 4-(1-ethoxyethenyl)-3,3,5,5-tetramethylcyclohexan-1-one |
| LILIAL | 80-54-6 | 3-(4-*tert*-butylphenyl)-2-methylpropanal |
| LINALYL ACETATE COEUR (drum) | 115-95-7 | 3,7-Dimethyl-1,6-octadien-3-yl acetate |
| MACROLIDE^{®} SUPRA | 106-02-5 | oxacyclohexadecan-2-one |
| MAJANTOL^{®} | 103694-68-4 | 2,2-dimethyl-3-(3-methylphenyl)propan-1-ol |
| METHYL ANTHRANILATE | 134-20-3 | Methyl-2-(amino)benzoate |
| METHYL NAPHTHYL KETONE BETA CRYST | 93-08-3 | Methyl-2-naphthyl ketone |
| METHYL SALICYLATE PF | 119-36-8 | methyl 2-hydroxybenzoate |
| MUGETANOL | 63767-86-2 | 1-(4-propan-2-ylcyclohexyl)ethanol |
| NEROLEX (drum) | 106-25-2 | (2Z)-3,7-dimethylocta-2,6-dien-1-ol |
| NEROLIDOL | 7212-44-4 | (6E)-3,7,11-trimethyldodeca-1,6,10-trien-3-ol |
| NEROLIN BROMELIA | 93-18-5 | 2-ethoxynaphthalene |
| NEROLIN YARA YARA CRIST. | 93-04-9 | 2-methoxynaphthalene |
| NEROLIONE | 23911-56-0 | 1-(3-methyl-1-benzofuran-2-yl)ethanone |
| NERYL ACETATE | 141-12-8 | [(2Z)-3,7-dimethylocta-2,6-dienyl] acetate |
| ORYCLON SPECIAL | 32210-23-4 | (4-tert-butylcyclohexyl) acetate |
| PATCHOULI OIL DECOL. | 84238-39-1 | ESSENTIAL OIL - PATCHOULOL |
| PEONILE | 10461-98-0 | 2-cyclohexylidene-2-phenylacetonitrile |
| PHENOXANOL | 55066-48-3 | 3-methyl-5-phenylpentan-1-ol |
| PROJASMONE P | 137-03-1 | 2-heptylcyclopentan-1-one |
| ROSAPHEN^{®} | 25634-93-9 | 2-methyl-5-phenylpentan-1 -ol |
| SANDRANOL^{®} | 106185-75-5 | (E)-2-ethyl-4-(2,2,3-trimethylcyclopent-3-en-1-yl)but-2-en-1-ol |
| STYRALYL ACETATE | 93-92-5 | 1-phenylethyl acetate |
| TETRAHYDRO MYRCENOL | 18479-57-7 | 2,6-Dimethyl-2-octanol |
| TETRAHYDROGERANIOL | 106-21-8 | 3,7-dimethyloctan-1-ol |
| VERTOFIX | 32388-55-9 | 1-[(1R,2R,5S,7R)-2,6,6,8-tetramethyl-9-tricyclo[5.3.1.01,5]undec-8-enyl]ethanone |
| VERTOMUGAL | 37677-14-8 | 4-(4-methylpent-3-enyl)cyclohex-3-ene-1-carbaldehyde |
| YSAMBER^{®} K | 154171-77-4 | (3'S,6'S,9'S)-2',2',8',8'-tetramethylspiro[1,3-dioxolane-2,10'-octahydro-1H-2,4a-methanonapthalene] |

Furthermore, the present invention also relates to a composition for depositing a fragrance on a fabric, comprising or consisting
of one or more fragrance compound(s) and at least one polyol ester of the formula (I) 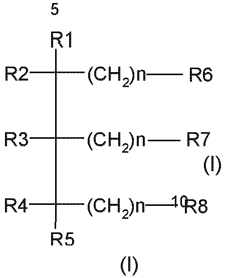
wherein R1, R2, R4 and R5 are independently selected from H, methyl and ethyl, and R3 is methyl or ethyl, preferably ethyl,
each n is independently 0, 1 or 2,
R6 to R8 are independently selected from H, OH, R9COO- and R10COO-,
wherein R9 is a branched or linear, saturated or unsaturated alkyl group with 8 to 10 carbon atoms, and
at least two of R6 to R8 are R10COO-,
wherein R10 is a linear, saturated alkyl with 8 to 10 carbon atoms,
preferably, wherein R9 is a linear, saturated or unsaturated alkyl group with 8 to 10 carbon atoms,
preferably, wherein R6 and R7 are R10COO- and R8 is R9COO-.

In one embodiment of the composition described above, in the polyol ester of formula (I) R1, R2, R4 and R5 are H.

In another embodiment of the composition according to any of the embodiments described above, two n are 0 and one n is 1.

In one embodiment of the composition according to any of the embodiments described above, R3 is CH₂CH₃.

In another embodiment of the composition according to any embodiment described above, the polyol ester has the formula (II) 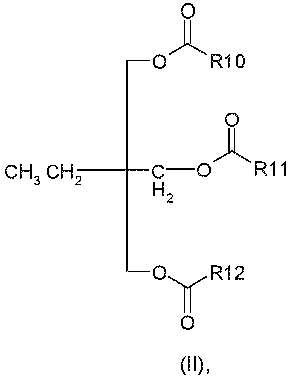 wherein R10 to R12 are independently selected from linear, saturated alkyl with 8 to 10 carbon atoms.

In a further embodiment of the composition according to any embodiment described above, the composition has a weight ratio of 50:50 or less polyol ester(s) to fragrance compound(s) or less, preferably 25:75 or less, particularly preferably 10:90 or less. In a preferred embodiment of the composition according to any embodiment described above, the composition has a weight ratio in a range of 9:91 to 1:99 polyol ester(s) to fragrance compound(s), preferably in a range of 6:94 to 1.5:98.5.

In one embodiment of the composition described above, the fragrance compound(s) is/are selected from aldehydes, ketones, esters, nitriles, ethers, lactones, primary alcohols, secondary alcohols, tertiary alcohols, phenolics, hydrocarbons, acetals and essential oils. In one embodiment of the composition according to any of the embodiments described above, the fragrance compound(s) is/are selected from the group consisting of compounds listed in Table 1.

The present invention also relates to a fabric comprising a composition according to any of the embodiments described above in contact with the fabric.

The composition according to the invention can advantageously be incorporated into a wide range of fabric rinse-off products.

The present invention therefore also relates to a product for depositing a fragrance to a fabric, comprising a composition according to any of the embodiments described above.

Preferably, the product is selected from the group consisting of fabric rinse-off products, such as laundry detergents, fabric softeners, fabric surface cleaning products, in particular aerosol surface spray and water based surface spray.

Finally, the present invention also relates to the use of a polyol ester, of the formula (I) 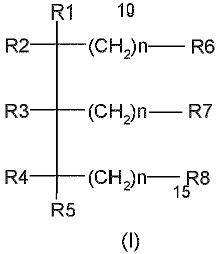
wherein R1, R2, R4 and R5 are independently selected from H, methyl and ethyl, and R3 is methyl or ethyl, preferably ethyl,
each n is independently 0, 1 or 2,
R6 to R8 are independently selected from H, OH, R9COO- and R10COO-,
wherein R9 is a branched or linear, saturated or unsaturated alkyl group with 8 to 10 carbon atoms, and
at least two of R6 to R8 are R10COO-,
wherein R10 is a linear, saturated alkyl with 8 to 10 carbon atoms,
preferably, wherein R9 is a linear, saturated or unsaturated alkyl group with 8 to 10 carbon atoms,
preferably, wherein R6 and R7 are R10COO-and R8 is R9COO-, for enhancing deposition of a fragrance on a fabric, in particular for increasing the retention of a fragrance on a fabric and/or for increasing fragrance strength on a fabric and/or for increasing fragrance quality on a fabric, preferably for increasing the retention of top, mid and base notes of a fragrance on a fabric and/or for increasing malodor counteraction activity of a fragrance on a fabric and/or for increasing antimicrobial activity of a fragrance on a fabric.

In one embodiment of the use described above, in the polyol ester of formula (I) R1, R2, R4 and R5 are H.

In another embodiment of the of the use according to any of the embodiments described above, two n are 0 and one n is 1.

In one embodiment of the of the use according to any of the embodiments described above, R3 is CH₂CH₃.

In a preferred embodiment of the use according to any of the embodiments described above, the polyol ester has the formula (II) 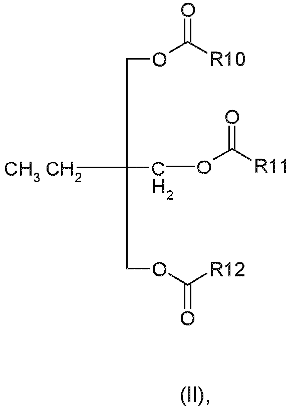 wherein R10 to R12 are independently selected from linear saturated alkyl with 8 to 10 carbon atoms.

In a further embodiment of the use according to any embodiment described above, the polyol ester is used at a weight ratio of 50:50 or less, preferably 25:75 or less, particularly preferably 10:90 or less with respect to the fragrance compound(s). In a preferred embodiment of the use according to any embodiment described above, the polyol ester is used at a range of 9:91 to 1:99, preferably in a range of 6:94 to 1.5:98.5 by weight with respect to the fragrance compound(s).

In a preferred embodiment of the use according to any of the embodiments described above, the fabric is selected from the group consisting of cotton and synthetic fabrics, in particular polyester, spandex, nylon and lycra and mixtures thereof.

In one embodiment of the use described above, the fragrance compound(s) is/are selected from aldehydes, ketones, esters, nitriles, ethers, lactones, primary alcohols, secondary alcohols, tertiary alcohols, phenolics, hydrocarbons, acetals and essential oils.

In one embodiment of the use according to any of the embodiments described above, the fragrance compound(s) is/are selected from the group consisting of the compounds given in Table 1.

### Short description of the figures:

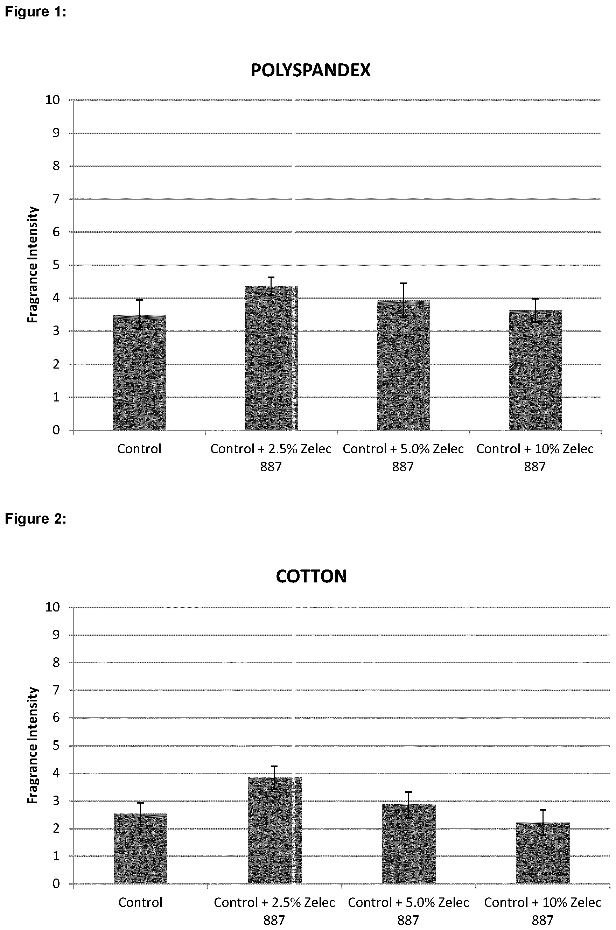

**Figure 1** shows the fragrance intensity on a scale from 0 to 10 of a control (a liquid fabric softener fragrance) with different doses of a polyol ester according to formula (II) (Zelec 887) on polyspandex after laundry and drying.
**Figure 2** shows the fragrance intensity on a scale from 0 to 10 of a control (a liquid fabric softener fragrance) with different doses of a polyol ester according to formula (II) (Zelec 887) on cotton after laundry and drying.

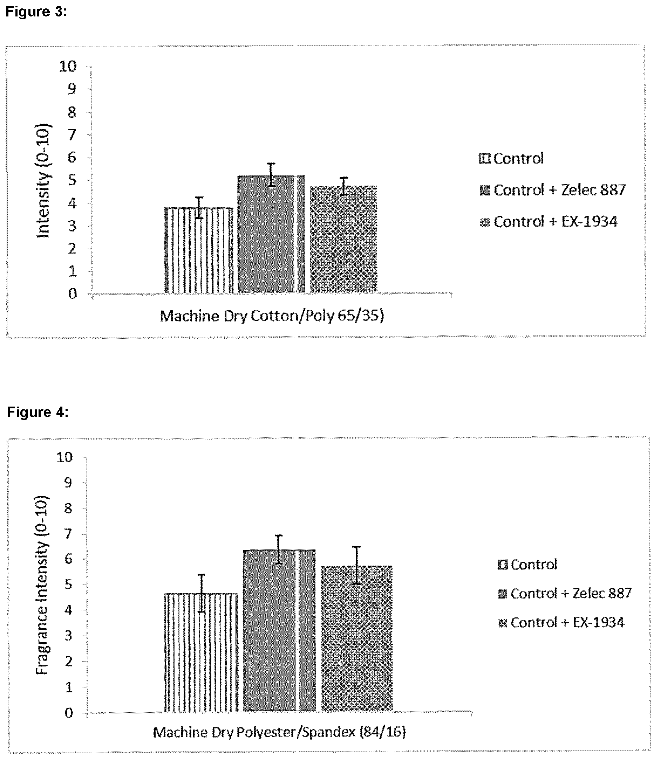

**Figure 3** shows the fragrance intensity on a scale from 0 to 10 of a control (a liquid laundry detergent fragrance, left column) with 2.5% of a polyol ester according to formula (II) (Zelec 887, middle column) or 2.5% of a polyol ester of formula (I) (EX-1934, right column) on a machine dried mixed fabric consisting of 65% cotton and 35% polyester. The fragrance intensities from left to right are 3.8, 5.2 and 4.7.
**Figure 4** shows the fragrance intensity on a scale from 0 to 10 of a control (a liquid laundry detergent fragrance, left column) with 2.5% of a polyol ester according to formula (II) (Zelec 887, middle column) or 2.5% of a polyol ester of formula (I) (EX-1934, right column) on a machine dried mixed fabric consisting of 84% polyester and 16% spandex. The fragrance intensities from left to right are 4.28, 6.83 and 6.22.

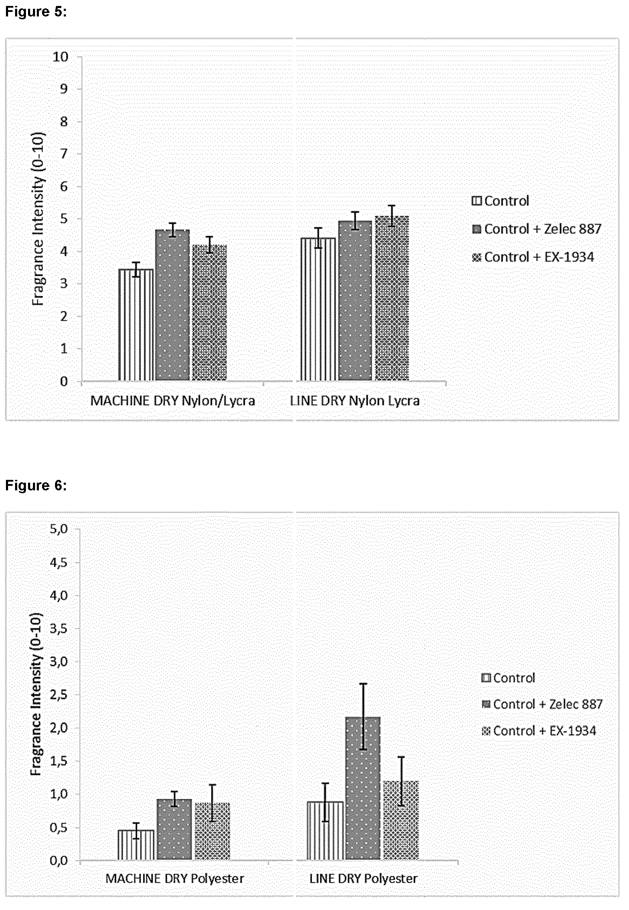

**Figure 5** shows the fragrance intensity on a scale from 0 to 10 of a control (a liquid laundry detergent fragrance, left column) with 2.5% of a polyol ester according to formula (II) (Zelec 887, middle column) or 2.5% of a polyol ester of formula (I) (EX-1934, right column) on a machine dried and line dried mixed fabric consisting of 88% nylon and 12% lycra. The fragrance intensities from left to right are 3.45, 4.67 and 4.21 for machine dried and 4.41, 4.94 and 5.09 for line dried.
**Figure 6** shows the fragrance intensity on a scale from 0 to 10 of a control (a liquid laundry detergent fragrance, left column) with 2.5% of a polyol ester according to formula (II) (Zelec 887, middle column) or 2.5% of a polyol ester of formula (I) (EX-1934, right column) on a machine dried and line dried polyester. The fragrance intensities from left to right are 0.45, 0.93 and 0.87 for machine dried and 0.88, 2.17 and 1.20 for line dried.

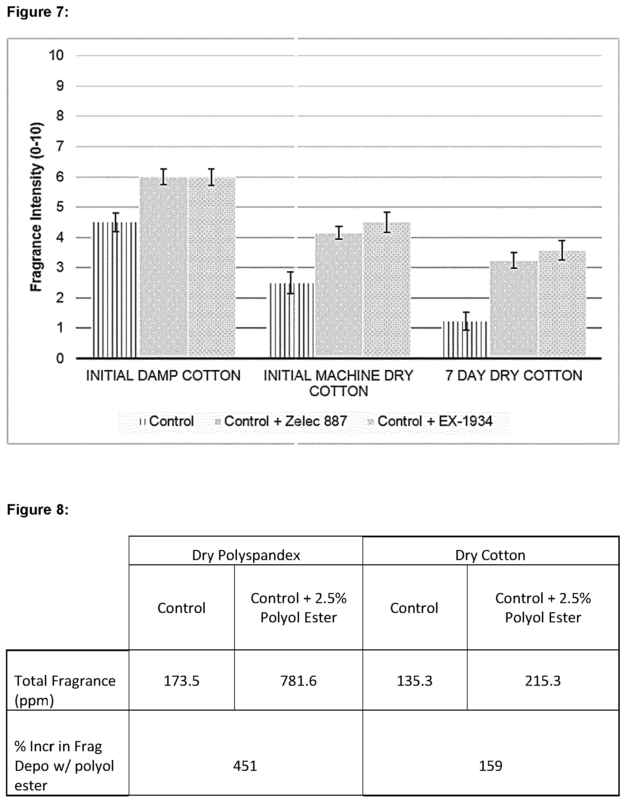

**Figure 7** shows the fragrance intensity on a scale from 0 to 10 of a control (a liquid fabric softener fragrance, left column) with 2.5% of a polyol ester according to formula (II) (Zelec 887, middle column) or 2.5% of a polyol ester of formula (I) (EX-1934, right column) on damp cotton, machine dried cotton and on cotton that has been dry for seven days. The fragrance intensities from left to right are 4.5, 6.0 and 6.0 for damp, 2.5, 4.2 and 4.5 for machine dried and 1.2, 3.2 and 3.6 for seven day dry.
**Figure 8** shows a comparison of the total fragrance that could be extracted from dry polyspandex and dry cotton after treatment with a control and the control with 2.5% of the polyol ester Zelec 887 and the increase in fragrance deposition with Zelec 887.

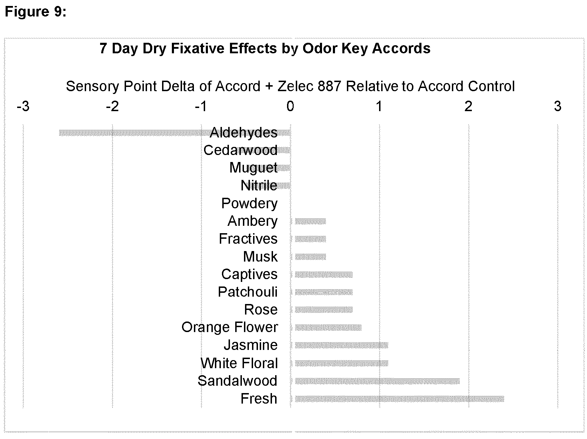

**Figure 9** shows the comparative testing of olfactive family accords with and without the inventive polyol ester. Perfume Raw Materials were selected and organized by odor key. The control was the accord of each individual odor key accord, the test sample contained the same accord plus the inventive polyol ester.

### Example 1: Initial testing

A liquid fabric softener fragrance as control was split into 4 aliquots to which increasing ratios (2.5%, 5% and 10%) of Zelec 887 (a polyol ester according to formula (II), commercially available from Stepan Company, Northbrook USA) were dosed. The control plus each Zelec 887-containing test sample were formulated at a total dosage of 1% (w/w) into a commercial liquid fabric softener (All Free & Clear). Each sample was laundered independently according to typical US laundering conditions for a fabric softener: 2 kg of fabric ballast, medium-sized cold wash water laundry load (10 minutes wash cycle) and one cold water rinse. 35 g of liquid fabric softener are dosed per laundry load. The entire fabric ballast was machine dried (high heat for 60 minutes) or line dried (24 hours on a drying rack). Polyspandex and cotton were used as fabrics and the fragrance intensity after laundry and drying was assessed. To this end, each test sample and control was blind coded with a random 3 digit number. Expert panelists use EyeQuestion Sensory software to rate the fragrance intensity of the blind-coded, randomized samples on a 0-10 linear scale. A minimum of 10 trained panelists was used to determine statistical significance. The results are shown in Figures 1 and 2. At a dosage of 2.5 % and 5 % polyol ester, a significant increase in fragrance intensity with respect to the control can be observed.

### Example 2: Liquid laundry detergent

Based on the initial testing in Example 1, a 2.5% dose (with respect to the fragrance composition) of Zelec 887 or EX-1934 (a polyol ester of formula (I), commercially available from Lubrizol Company, Wickliffe, USA) were tested accordingly with a commercial liquid laundry detergent on a range of fabric types. The results are shown in Figures 3 to 6. The combination with a polyol ester lead to an increase in fragrance intensity.

### Example 3: Liquid fabric softener

In order to determine the longevity of fragrance retention on cotton, a liquid fabric softener with 2.5 % polyol ester (with respect to the fragrance composition) and without polyol ester was used under the same conditions as described in Example 1. After laundering and drying, the cotton was stored for seven days. Since the use of fabric softener is not recommended on synthetic fabrics, this experiment was done only with cotton. The results are shown in Figure 7. At all stages, initial damp cotton, machine dried cotton and seven day dry cotton, the fragrance intensity was larger in the presence of polyol esters Zelec 887 and Ex-1934 compared to the control.

### Example 4: Analytical extractions

Fragrance ingredients from the fragrance composition used in Examples 1 to 3 were quantified on a laundered polyspandex and cotton towel for a control and for the control with 2.5% Zelec 887 (with respect to the fragrance composition). This was done with industry standard solvent extraction of the towel to strip off the fragrance ingredients, a standard was added at a known concentration and based on the GC/MS area response of the standard, the analytes (fragrance ingredients) were quantified. The list of fragrance ingredients shows that for each sample, which contains Zelec 887, the concentration of each fragrance ingredient on the towel is greater than for the control. The results are shown in Tables 2 and 3 and summarized in Figure 8. The GC/MS output is a peak when a fragrance ingredient is detected. The area counts are the area under the peak for that unique fragrance ingredient and the total area under that peak is linearly proportional to its concentration. In this experimental set, the concentration of the Internal standard (IS), Phenetole, is 100 ppm. The signal from phenetole is 18453977 area counts. Ingredient Concentration Mathematical Expression: (Phenteole Concentration / Phenetole Area) x Ingredient Area = Ingredient Concentration. The tables show that more fragrance was deposited and extracted from the polyol ester test sample than the control (without polyol esters). The increase in fragrance concentration on the fabrics is directly correlated to increased fragrance intensity as represented by the sensory performance data.

**Table 2: Fragrance Ingredients quantified on polyspandex towel**

| **4" x 4" Polyspandex swatch (Liquid Laundry Detergent)** | | | | | |
|---|---|---|---|---|---|
| **Polyol Ester** | | | **Control** | | |
| **Ingredient** | **Peak Area** | **Conc (ppm)** | **Ingredient** | **Peak Area** | **Conc (ppm)** |
| Phenetole (IS) | 18453977 | 100.0 | Phenetole (IS) | 36668876 | 93.0 |
| Iraldeine Gamma | 3414443 | 18.5 | Iraldeine Gamma | 2436860 | 6.6 |
| Herbaflorat | 57077419 | 309.3 | Herbaflorat | 12728556 | 34.7 |
| Ebanol | 1227752 | 6.7 | Ebanol | 0 | 0.0 |
| Floralozone | 1114168 | 6.0 | Floralozone | 0 | 0.0 |
| Herbyl Propionate | 2146923 | 11.6 | Herbyl Propionate | 0 | 0.0 |
| Cyclamen Aldehyde | 8367029 | 45.3 | Cyclamen Aldehyde | 5745989 | 15.7 |
| Lilial | 28843718 | 156.3 | Lilial | 18446144 | 50.3 |
| Iso E Super | 10338787 | 56.0 | Iso E Super | 5721932 | 15.6 |
| Nerolin Yara Yara | 11622955 | 63.0 | Nerolin Yara Yara | 6320000 | 17.2 |
| Hedione | 6060870 | 32.8 | Hedione | 2995247 | 8.2 |
| Hexylcinnamaldehy de | 3510340 | 19.0 | Hexylcinnamaldehy de | 2511422 | 6.8 |
| Globanone | 3007668 | 16.3 | Globanone | 1728587 | 4.7 |
| Peonile | 7509775 | 40.7 | Peonile | 4968007 | 13.5 |
| **TOTAL:** | | **781.6** | **TOTAL:** | | **173.5** |

**Table 3: Fragrance Ingredients quantified on cotton towel**

| **12" x 12" 100% Cotton Towel (Liquid Fabric Softener Application)** | | | | | |
|---|---|---|---|---|---|
| **Polyol Ester** | | | **Control** | | |
| **FRM** | **Area Counts** | **Conc (ppm)** | **FRM** | **Area Counts** | **Conc (ppm)** |
| Phenetole (IS) | 6070300 | 100 | Phenetole (IS) | 5643784 | 75 |
| Lilial | 3335904 | 55.0 | Lilial | 2320789 | 30.8 |
| Iso E Super | 796287 | 13.1 | Iso E Super | 683139 | 9.1 |
| Nerolin Yara Yara | 206536 | 3.4 | Nerolin Yara Yara | 125457 | 1.7 |
| Isobutyl quinoline | 809865 | 13.3 | Isobutyl quinoline | 577134 | 7.7 |
| Hexylcinnamaldeh yde | 1852332 | 30.5 | Hexylcinnamaldeh yde | 830172 | 11.0 |
| **TOTAL:** | | **215.3** | **TOTAL:** | | **135.3** |

### Example 5:

Results on the comparative testing of olfactive family accords with and without the inventive polyol ester is shown in Figure 9. Perfume Raw Materials were selected and organized by odor key. The control was the accord of each individual odor key accord, the test sample contained the same accord plus the inventive polyol ester. Each sample was laundered independently according to typical US laundering conditions for a fabric softener: 2 kg of fabric ballast, medium-sized cold wash water laundry load (10 minutes wash cycle) and one cold water rinse. 35 g of liquid fabric softener are dosed per laundry load. The entire fabric ballast was machine dried (high heat for 60 minutes). Nylon/Lycra blend was used as fabric and the fragrance intensity after laundering cycle was assessed. To this end, each test sample and control was blind coded with a random 3 digit number. Expert panelists used EyeQuestion Sensory software to rate the fragrance intensity of the blind-coded, randomized samples on a 0-10 linear scale. The test was repeated at initial and 7 day dry time points. A minimum of 10 trained panelists were used to determine statistical significance.

## Claims

1. Method for imparting a fragrance to a fabric comprising or consisting of the following steps:
(i) providing a composition comprising or consisting of one or more fragrance compound(s) and at least one polyol ester of the formula (I), 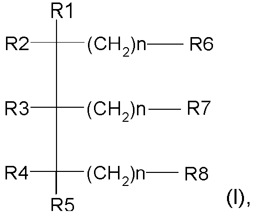
wherein R1, R2, R4 and R5 are independently selected from H, methyl and ethyl, and R3 methyl or ethyl, preferably ethyl,
each n is independently 0, 1 or 2,
R6 to R8 are independently selected from H, OH, R9COO- and R10COO-,
wherein R9 is a branched or linear, saturated or unsaturated alkyl group with 8 to 10 carbon atoms, and
at least two of R6 to R8 are R10COO-,
wherein R10 is a linear, saturated alkyl with 8 to 10 carbon atoms,
preferably, wherein R9 is a linear, saturated or unsaturated alkyl group with 8 to 10 carbon atoms,
preferably, wherein R6 and R7 are R10COO- and R8 is R9COO-,
(ii) contacting the composition from step (i) with the fabric, and
(iii) optionally, drying the fabric.

2. Method according to claim 1, wherein the polyol ester has the formula (II) 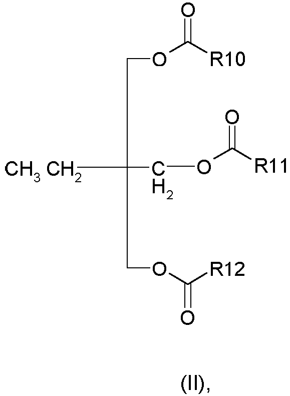 wherein R10 to R12 are independently selected from linear, saturated alkyl with 8 to 10 carbon atoms.

3. Method according to claim 1 or 2, wherein the fabric is selected from the group consisting of cotton and synthetic fabrics, in particular polyester, spandex, nylon and lycra.

4. Method according to claim 3, wherein the composition additionally imparts conditioning and/or softening effects on the fabric.

5. Method according to any of the preceding claims, wherein the composition provided in step (i) has a weight ratio of 50:50 or less polyol ester(s) to fragrance compound(s), preferably 25:75 or less, particularly preferably 10:90 or less, in particular the composition provided in step (i) has a weight ratio in a range of 9:91 to 1:99 polyol ester(s) to fragrance compound(s), preferably in a range of 6:94 to 1.5:98.5.

6. Method according to any of the preceding claims, wherein the fragrance compound(s) is/are selected from the group consisting of compounds listed in Table 1 of the description.

7. Composition for depositing a fragrance on a fabric, comprising or consisting of one or more fragrance compound(s) and at least one polyol ester of the formula (I) 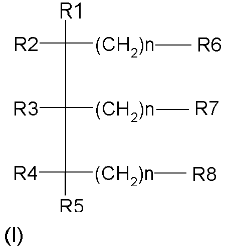
wherein R1, R2, R4 and R5 are independently selected from H, methyl and ethyl, and R3 is methyl or ethyl, preferably ethyl,
each n is independently 0, 1 or 2,
R6 to R8 are independently selected from H, OH, R9COO- and R10COO-,
wherein R9 is a branched or linear, saturated or unsaturated alkyl group with 8 to 10 carbon atoms, and
at least two of R6 to R8 are R10COO-,
wherein R10 is a linear, saturated alkyl with 8 to 10 carbon atoms,
preferably, wherein R9 is a linear, saturated or unsaturated alkyl group with 8 to 10 carbon atoms,
preferably, wherein R6 and R7 are R10COO- and R8 is R9COO-.

8. Composition according to claim 7, wherein the composition has a weight ratio of 50:50 or less polyol ester(s) to fragrance compound(s), preferably 25:75 or less, particularly preferably 10:90 or less, in particular the composition has a weight ratio in a range of 9:91 to 1:99 polyol ester(s) to fragrance compound(s), preferably in a range of 6:94 to 1.5:98.5.

9. Composition according to any of claims 7 to 8, wherein the fragrance compound(s) is/are selected from the group consisting of compounds listed in Table 1 of the description.

10. A fabric comprising a composition of any of claims 7 to 9 in contact with the fabric.

11. Product for depositing a fragrance to a fabric, comprising a composition according to any of claims 7 to 9.

12. Product according to claim 11, wherein the product is selected from the group consisting of fabric rinse-off products, such as laundry detergents, fabric softeners, fabric surface cleaning products, in particular aerosol surface spray and water based surface spray.

13. Use of a polyol ester, of the formula (I), 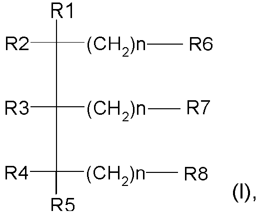
wherein R1, R2, R4 and R5 are independently selected from H, methyl and ethyl, and R3 is methyl or ethyl, preferably ethyl,
each n is independently 0, 1 or 2,
R6 to R8 are independently selected from H, OH, R9COO- and R10COO-,
wherein R9 is a branched or linear, saturated or unsaturated alkyl group with 8 to 10 carbon atoms, and
at least two of R6 to R8 are R10COO-,
wherein R10 is a linear, saturated alkyl with 8 to 10 carbon atoms, preferably, wherein R9 is a linear, saturated or unsaturated alkyl group with 8 to 10 carbon atoms,
preferably, wherein R6 and R7 are R10COO- and R8 is R9COO-,
for enhancing deposition of a fragrance on a fabric, in particular for increasing the retention of a fragrance on a fabric and/or for increasing fragrance strength on a fabric and/or for increasing fragrance quality on a fabric, preferably for increasing the retention of top, mid and base notes of a fragrance on a fabric and/or for increasing malodor counteraction activity of a fragrance on a fabric and/or for increasing antimicrobial activity of a fragrance on a fabric.

14. Use according to claim 13, wherein the polyol ester has the formula (II) 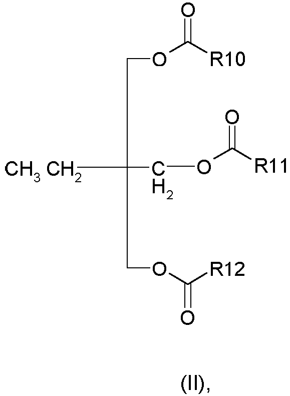 wherein R10 to R12 are independently selected from linear saturated alkyl with 8 to 10 carbon atoms.

15. Use according to claim 13 or 14, wherein the fabric is selected from the group consisting of cotton and synthetic fabrics, in particular polyester, spandex, nylon and lycra and mixtures thereof and/or wherein the fragrance compound(s) is/are selected from the group consisting of compounds listed in Table 1 of the description.

## Patentansprüche

1. Verfahren zur Verleihung eines Duftes an ein Gewebe, umfassend oder bestehend aus den folgenden Schritten:
(i) Bereitstellen einer Zusammensetzung, die eine oder mehrere Duftstoffverbindungen und mindestens einen Polyolester der Formel (I) umfasst oder daraus besteht, 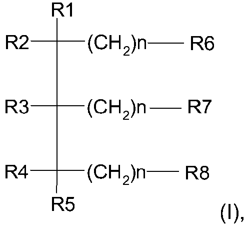
wobei R1, R2, R4 und R5 unabhängig voneinander ausgewählt sind aus H, Methyl und Ethyl, und R3 Methyl oder Ethyl, vorzugsweise Ethyl ist,
jedes n unabhängig 0, 1 oder 2 ist,
R6 bis R8 unabhängig voneinander ausgewählt sind aus H, OH, R9COO- und R10COO-,
wobei R9 eine verzweigte oder lineare, gesättigte oder ungesättigte Alkylgruppe mit 8 bis 10 Kohlenstoffatomen ist, und
mindestens zwei von R6 bis R8 R10COO- sind,
wobei R10 ein linearer, gesättigter Alkylrest mit 8 bis 10 Kohlenstoffatomen ist,
vorzugsweise, wobei R9 eine lineare, gesättigte oder ungesättigte Alkylgruppe mit 8 bis 10 Kohlenstoffatomen ist,
vorzugsweise, wobei R6 und R7 R10COO- sind und R8 R9COO- ist,
(ii) In-Kontakt-Bringen der Zusammensetzung aus Schritt (i) mit dem Gewebe, und
(iii) optionales Trocknen des Gewebes.

2. Verfahren nach Anspruch 1, wobei der Polyolester die Formel (II) hat: 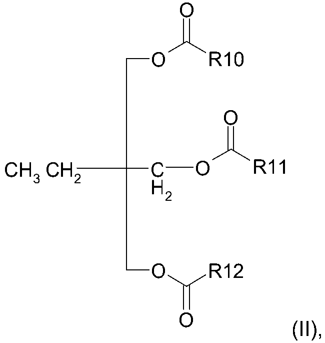 wobei R10 bis R12 unabhängig voneinander ausgewählt sind aus linearen, gesättigten Alkylresten mit 8 bis 10 Kohlenstoffatomen.

3. Verfahren nach Anspruch 1 oder 2, wobei das Gewebe ausgewählt ist aus der Gruppe bestehend aus Baumwolle und synthetischen Geweben, insbesondere Polyester, Spandex, Nylon und Lycra.

4. Verfahren nach Anspruch 3, wobei die Zusammensetzung zusätzlich konditionierende und/oder weichmachende Effekte auf das Gewebe verleiht.

5. Verfahren nach einem der vorstehenden Ansprüche, wobei die in Schritt (i) bereitgestellte Zusammensetzung ein Gewichtsverhältnis von 50:50 oder weniger Polyolester zu Duftstoffverbindung(en) aufweist, vorzugsweise 25:75 oder weniger, besonders bevorzugt 10:90 oder weniger, insbesondere die in Schritt (i) bereitgestellte Zusammensetzung ein Gewichtsverhältnis im Bereich von 9:91 bis 1:99 Polyolester zu Duftstoffverbindung(en), vorzugsweise im Bereich von 6:94 bis 1,5:98,5 aufweist.

6. Verfahren nach einem der vorstehenden Ansprüche, wobei die Duftstoffverbindung(en) ausgewählt ist/sind aus der Gruppe bestehend aus den in Tabelle 1 der Beschreibung aufgeführten Verbindungen.

7. Zusammensetzung zur Ablagerung eines Duftes auf einem Gewebe, umfassend oder bestehend aus einer oder mehreren Duftstoffverbindung(en) und mindestens einem Polyolester der Formel (I), 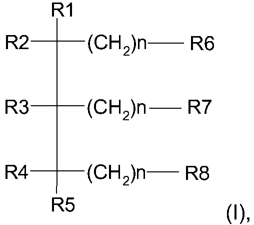
wobei R1, R2, R4 und R5 unabhängig voneinander ausgewählt sind aus H, Methyl und Ethyl, und R3 Methyl oder Ethyl, vorzugsweise Ethyl ist,
jedes n unabhängig 0, 1 oder 2 ist,
R6 bis R8 unabhängig voneinander ausgewählt sind aus H, OH, R9COO- und R10COO-,
wobei R9 eine verzweigte oder lineare, gesättigte oder ungesättigte Alkylgruppe mit 8 bis 10 Kohlenstoffatomen ist, und
mindestens zwei von R6 bis R8 R10COO- sind,
wobei R10 ein linearer, gesättigter Alkylrest mit 8 bis 10 Kohlenstoffatomen ist,
vorzugsweise, wobei R9 eine lineare, gesättigte oder ungesättigte Alkylgruppe mit 8 bis 10 Kohlenstoffatomen ist,
vorzugsweise, wobei R6 und R7 R10COO- sind und R8 R9COO- ist.

8. Zusammensetzung nach Anspruch 7, wobei die Zusammensetzung ein Gewichtsverhältnis von 50:50 oder weniger Polyolester zu Duftstoffverbindung(en) aufweist, vorzugsweise 25:75 oder weniger, besonders bevorzugt 10:90 oder weniger, insbesondere die Zusammensetzung ein Gewichtsverhältnis im Bereich von 9:91 bis 1:99, vorzugsweise im Bereich von 6:94 bis 1,5:98,5 Polyolester zu Duftstoffverbindung(en) aufweist.

9. Zusammensetzung nach einem der Ansprüche 7 bis 8, wobei die Duftstoffverbindung(en) ausgewählt ist/sind aus der Gruppe bestehend aus den in Tabelle 1 der Beschreibung aufgeführten Verbindungen.

10. Gewebe, das eine Zusammensetzung nach einem der Ansprüche 7 bis 9 in Kontakt mit dem Gewebe umfasst.

11. Produkt zur Ablagerung eines Duftes auf einem Gewebe, umfassend eine Zusammensetzung nach einem der Ansprüche 7 bis 9.

12. Produkt nach Anspruch 11, wobei das Produkt ausgewählt ist aus der Gruppe bestehend aus Gewebe-Spülprodukten, wie Waschmitteln, Weichspülern, Gewebeoberflächen-Reinigungsprodukten, insbesondere Aerosol-Oberflächensprays und wasserbasierten Oberflächensprays.

13. Verwendung eines Polyolesters der Formel (I), 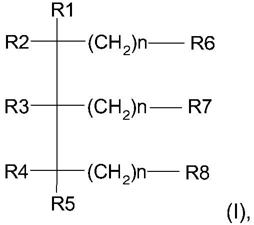
wobei R1, R2, R4 und R5 unabhängig voneinander ausgewählt sind aus H, Methyl und Ethyl, und R3 Methyl oder Ethyl, vorzugsweise Ethyl ist,
jedes n unabhängig 0, 1 oder 2 ist,
R6 bis R8 unabhängig voneinander ausgewählt sind aus H, OH, R9COO- und R10COO-,
wobei R9 eine verzweigte oder lineare, gesättigte oder ungesättigte Alkylgruppe mit 8 bis 10 Kohlenstoffatomen ist, und
mindestens zwei von R6 bis R8 R10COO- sind,
wobei R10 ein linearer, gesättigter Alkylrest mit 8 bis 10 Kohlenstoffatomen ist,
vorzugsweise, wobei R9 eine lineare, gesättigte oder ungesättigte Alkylgruppe mit 8 bis 10 Kohlenstoffatomen ist,
vorzugsweise, wobei R6 und R7 R10COO- sind und R8 R9COO- ist,
zur Verbesserung der Ablagerung eines Duftes auf einem Gewebe, insbesondere zur Erhöhung der Retention eines Duftes auf einem Gewebe und/oder zur Erhöhung der Duftintensität auf einem Gewebe und/oder zur Verbesserung der Duftqualität auf einem Gewebe, vorzugsweise zur Erhöhung der Retention von Kopf-, Herz- und Basisnoten eines Duftes auf einem Gewebe und/oder zur Erhöhung der Geruchsüberdeckungsaktivität eines Duftes auf einem Gewebe und/oder zur Erhöhung der antimikrobiellen Aktivität eines Duftes auf einem Gewebe.

14. Verwendung nach Anspruch 13, wobei der Polyolester die Formel (II) hat: 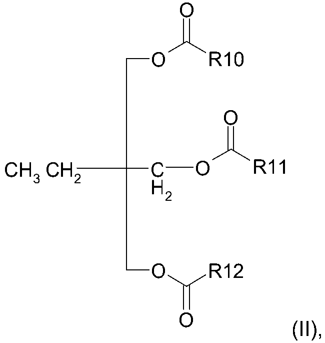 wobei R10 bis R12 unabhängig voneinander ausgewählt sind aus linearen, gesättigten Alkylresten mit 8 bis 10 Kohlenstoffatomen.

15. Verwendung nach Anspruch 13 oder 14, wobei das Gewebe ausgewählt ist aus der Gruppe bestehend aus Baumwolle und synthetischen Geweben, insbesondere Polyester, Spandex, Nylon und Lycra und deren Mischungen und/oder wobei die Duftstoffverbindung(en) ausgewählt ist/sind aus der Gruppe bestehend aus den in Tabelle 1 der Beschreibung aufgeführten Verbindungen.

## Revendications

1. Procédé destiné à conférer un parfum à un tissu, comprenant les ou constitué des étapes suivantes consistant à:
(i) fournir une composition comprenant ou constituée (d')un ou (de) plusieurs composé(s) de parfum et (d')au moins un ester de polyol de formule (I). 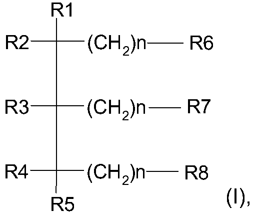
dans laquelle R1, R2, R4 et R5 sont indépendamment choisis parmi H, méthyle et éthyle, et R3 représente du méthyle ou de l'éthyle, de préférence de l'éthyle,
chaque n est indépendamment égal à 0, 1 ou 2,
R6 à R8 sont indépendamment choisis parmi H, OH, R9COO- et R10COO-,
dans laquelle R9 représente un groupe alkyle ramifié ou linéaire, saturé ou insaturé, comprenant 8 à 10 atomes de carbone, et
au moins deux des R6 à R8 représentent R10COO-,
dans laquelle R10 représente un radical alkyle linéaire, saturé comprenant 8 à 10 atomes de carbone,
de préférence, dans laquelle R9 représente un groupe alkyle linéaire, saturé ou insaturé, comprenant 8 à 10 atomes de carbone,
de préférence, dans laquelle R6 et R7 représentent R10COO- et R8 représente R9COO-,
(ii) mettre en contact la composition de l'étape (i) avec le tissu, et
(iii) en option, sécher le tissu.

2. Procédé selon la revendication 1, dans lequel l'ester de polyol présente la formule (II) 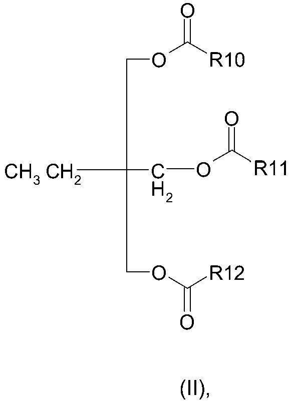 dans laquelle R10 à R12 sont indépendamment choisis parmi des radicaux alkyles linéaires saturés comprenant 8 à 10 atomes de carbone.

3. Procédé selon la revendication 1 ou 2, dans lequel le tissu est choisi dans le groupe constitué par le coton et les tissus synthétiques, en particulier le polyester, l'élasthanne, le nylon et le lycra.

4. Procédé selon la revendication 3, dans lequel la composition confère en outre des effets conditionnants et/ou adoucissants au tissu.

5. Procédé selon l'une quelconque des revendications précédentes, dans lequel la composition fournie à l'étape (i) présente un rapport pondéral ester(s) de polyol/composé(s) de parfum de 50 : 50 ou moins, de préférence de 25 : 75 ou moins, de manière particulièrement préférée de 10 : 90 ou moins, en particulier, la composition fournie à l'étape (i) présente un rapport pondéral ester(s) de polyol/composé(s) de parfum compris entre 9 : 91 et 1 : 99, de préférence compris entre 6 : 94 et 1,5 : 98,5.

6. Procédé selon l'une quelconque des revendications précédentes, dans lequel le(s) composé(s) de parfum est/sont choisi(s) dans le groupe constitué par des composés listés dans le tableau 1 de la description.

7. Composition pour déposer un parfum sur un tissu, comprenant ou constituée (d')un ou (de) plusieurs composés de parfum et (d')au moins un ester de polyol de formule (I), 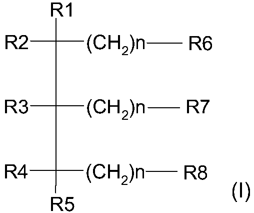
dans laquelle R1, R2, R4 et R5 sont indépendamment choisis parmi H, méthyle et éthyle, et R3 représente du méthyle ou de l'éthyle, de préférence de l'éthyle,
chaque n est indépendamment égal à 0, 1 ou 2,
R6 à R8 sont indépendamment choisis parmi H, OH, R9COO- et R10COO-,
dans laquelle R9 est un groupe alkyle ramifié ou linéaire, saturé ou insaturé, comprenant 8 à 10 atomes de carbone, et
au moins deux des R6 à R8 représentent R10COO-,
dans laquelle R10 représente un radical alkyle linéaire, saturé comprenant 8 à 10 atomes de carbone,
de préférence, dans laquelle R9 est un groupe alkyle linéaire, saturé ou insaturé, comprenant 8 à 10 atomes de carbone,
de préférence, dans laquelle R6 et R7 représentent R10COO- et R8 représente R9COO-.

8. Composition selon la revendication 7, dans laquelle la composition présente un rapport pondéral ester(s) de polyol/composé(s) de parfum de 50 : 50 ou moins, de préférence de 25 : 75 ou moins, de manière particulièrement préférée de 10 : 90 ou moins, en particulier, la composition présente un rapport pondéral ester(s) de polyol/composé(s) de parfum compris entre 9 : 91 et 1 : 99, de préférence compris entre 6 : 94 et 1,5 : 98,5.

9. Composition selon l'une quelconque des revendications 7 ou 8, dans laquelle le(s) composé(s) de parfum est/sont choisi(s) dans le groupe constitué par des composés listés dans le tableau 1 de la description.

10. Tissu comprenant une composition selon l'une quelconque des revendications 7 à 9, en contact avec le tissu.

11. Produit pour déposer un parfum sur un tissu, comprenant une composition selon l'une quelconque des revendications 7 à 9.

12. Produit selon la revendication 11, dans lequel le produit est choisi dans le groupe constitué par les produits de rinçage de tissu, tels que les lessives, les assouplissants, les produits de nettoyage de surface de tissu, en particulier les sprays de surface en aérosol et les sprays de surface à base d'eau.

13. Utilisation d'un ester de polyol, de formule (I), 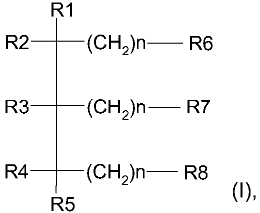
dans laquelle R1, R2, R4 et R5 sont indépendamment choisis parmi H, méthyle et éthyle, et R3 représente du méthyle ou de l'éthyle, de préférence de l'éthyle,
chaque n est indépendamment égal à 0, 1 ou 2,
R6 à R8 sont indépendamment choisis parmi H, OH, R9COO- et R10COO-,
dans laquelle R9 représente un groupe alkyle ramifié ou linéaire, saturé ou insaturé, comprenant 8 à 10 atomes de carbone, et
au moins deux des R6 à R8 représentent R10COO-,
dans laquelle R10 représente un radical alkyle linéaire, saturé comprenant 8 à 10 atomes de carbone,
de préférence, dans laquelle R9 représente un groupe alkyle linéaire, saturé ou insaturé, comprenant 8 à 10 atomes de carbone,
de préférence, dans laquelle R6 et R7 représentent R10COO- et R8 représente R9COO-,
pour améliorer le dépôt d'un parfum sur un tissu, en particulier pour augmenter la rétention d'un parfum sur un tissu et/ou pour augmenter l'intensité de parfum sur un tissu et/ou pour augmenter la qualité de parfum sur un tissu, de préférence pour augmenter la rétention de notes de tête, de cœur et de fond d'un parfum sur un tissu et/ou pour augmenter l'activité anti-mauvaises odeurs d'un parfum sur un tissu et/ou pour augmenter l'activité antimicrobienne d'un parfum sur un tissu.

14. Utilisation selon la revendication 13, dans laquelle l'ester de polyol présente la formule (II) 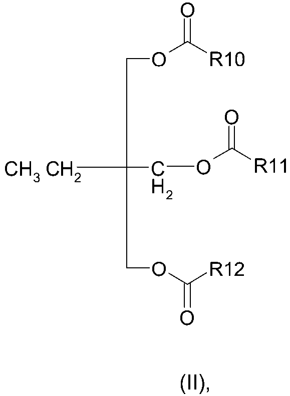 dans laquelle R10 à R12 sont indépendamment choisis parmi des radicaux alkyles linéaires saturés comprenant 8 à 10 atomes de carbone.

15. Utilisation selon la revendication 13 ou 14, dans laquelle le tissu est choisi dans le groupe constitué par le coton et les tissus synthétiques, en particulier le polyester, l'élasthanne, le nylon et le lycra et leurs mélanges et/ou dans laquelle le(s) composé(s) de parfum est/sont choisi(s) dans le groupe constitué par des composés listés dans le tableau 1 de la description.
